# EUROPEAN PATENT SPECIFICATION

(11) **EP 2 928 434 B1**
(45) Date of publication and mention of the grant of the patent: **09.08.2017**
(21) Application number: 13863657.6
(22) Date of filing: 04.12.2013
(51) Int. Cl.: A61F 15/00

(54) **UNIVERSAL LOCKING CLIP**
UNIVERSELLE VERSCHLUSSKLAMMER
ATTACHE DE VERROUILLAGE UNIVERSELLE

(30) Priority: 10.12.2012 US 201261735436 P; 14.03.2013 US 201361783672 P
(43) Date of publication of application: 14.10.2015
(73) Proprietor: 3M Innovative Properties Company, St. Paul, MN 55133-3427 (US)
(72) Inventor: JULIAN, Dominic J., Saint Paul, Minnesota 55133-3427 (US); JACOBS, Jeremy J., Saint Paul, Minnesota 55133-3427 (US)
(74) Representative: Vossius & Partner Patentanwälte Rechtsanwälte mbB
(86) International application number: PCT/US2013/073000
(87) International publication number: WO 2014/093077

(56) References cited:
- CH-A- 359 241
- GB-A- 1 505 288
- US-A- 2 820 456
- US-A- 3 036 572
- US-A- 3 307 233
- US-A- 3 373 742
- US-A- 3 857 140
- US-A- 3 863 301
- US-A- 4 586 499

## Description

### Background

Elastic bandages, including those offered under the ACE™ brand, are particular useful for providing short term compression and minor immobilization for human and animal anatomy. Application of these bandages can employ figure eight pattern type wrapping and the bandages are commonly wrapped in a continuous manner. In wrapping a user, it is common practice to initially have the bandage rolled neatly and to wrap the bandage around a portion of the body as the bandage is being unrolled. In order to apply the bandages in this fashion, the bandages are usually of a considerable length such as four feet or greater. Elasticized bandages and more specifically elastic bandages which are elastic in the longitudinal direction present significant advantages over their non-elastic counterparts. These advantages include an increased ability to accommodate the curvature of body parts, improved ability to stay on without slipping, the ability to apply a relatively even amount of bandage pressure useful for the treatment of ailments and wounds, and the ability to wrap a portion of the body using a significantly shorter and hence a lighter bandage.

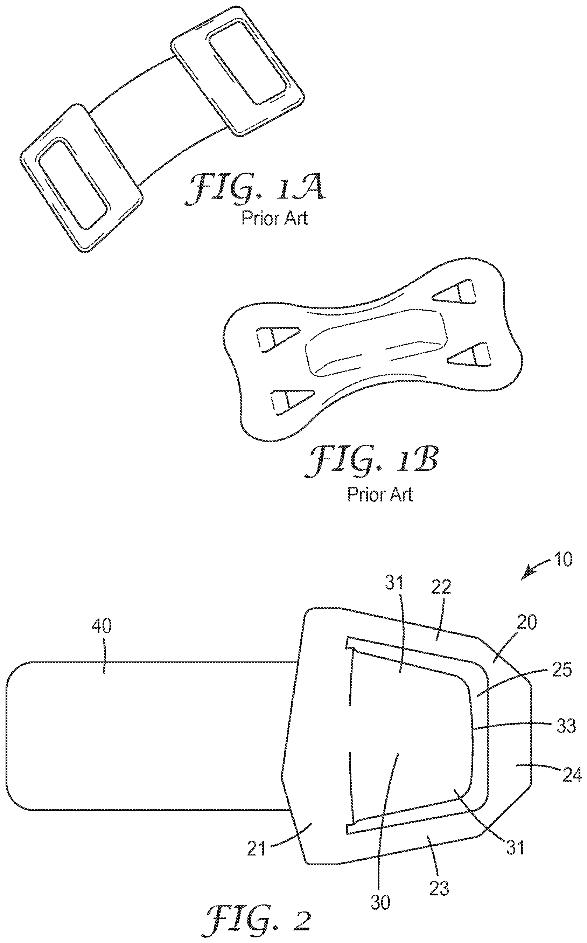

Elastic bandages are typically retained in a wrapped position by one or more metal clips, as disclosed in e.g. US 3,307,233 or US 3,372,742. The metal clips have relatively sharp projections (i.e., prongs) formed thereon which, when in use, pierce the end of the bandage and also pierce an intermediate portion of the bandage thereby holding the end of the bandage in contact with the intermediate portion of the bandage (see Figure 1). Other retaining system include hook and loop fastening (e.g., those incorporating Velcro™ brand products) capability, or those disclosed in GB 1 505 288, US 3,857,140 or CH 359241.

### Summary

The securement of the prong-style clips to elastic bandages has proven challenging, with the added risk of the prongs poking the user as they pierce through the elastic material. This can be a problem for a user trying to place the bandage on her own body. The prong-style clips also exhibit a tendency to fall off and get lost or misplaced when not in use. The more recent alternative, the use of a hook and loop fastening system (e.g., Velcro™) that is secured or integral with a portion of the wrap, provides some improvement over the prong system, but can be costly to manufacture and can limit user customization. Thus, there remains a need for innovative means to secure disparate portions of a textile to provide sufficient performance when worn by a user.

The present disclosure provides a clip having a body defining a holding area, a tongue, and a fastening strap. The body and tongue cooperate to secure a segment of a target material, while the fastening strap can adhere, preferably removably, to another segment thereof.

During use, the tongue may be moved (e.g., depressed) relative to the body. Alternatively, the body may be moved relative to the tongue. The target material (e.g., an elastic wrap) is inserted into the holding area and the tongue (or body) is again moved relative to its initial position to frictionally engage the body (or tongue). The body provides a bridge or ledge-like surface for the tongue to engage and remain, securing the target material in place. The frictional engagement provides a "snapping feature", an audible tone that helps the user ensure that it has been properly secured to the elastic wrap. The snapping feature also provides tactile feedback and a pinch-point to secure the material. The clip also can to be secured to and removed from a textile material like an elastic wrap without damage or puncture of the material. Additionally, the clips of the present disclosure can be removed and secured at various points along the elastic bandage (or other target material), including the mid-line or any other locations deemed necessary for best results and/or wearer preference.

The clip can be applied to any sort of a strap or strip of material utilizing various suitable attachment mechanisms, including but not limited to adhesives, gels, and micro hooks. The clip can also be utilized in the opposite manner, where the fastener can be the primary point of engagement (e.g., sewn in place) and the clip portion utilized for final securement.

In one aspect, the present disclosure provides a clip comprising: a body including a base, a first arm, a second arm, and a bridge, the body defining a holding plane; a tongue projecting from the base in a direction relative to the holding plane; and a strap coupled to the body, wherein the tongue is operable to frictionally engage the bridge to secure a target material between the tongue and the bridge.

In another aspect, the present disclosure provides a method for securing a first segment of an elastic wrap to a second segment of the elastic wrap, the method comprising: providing a clip including a body, a tongue having a tip, and a fastening strap, wherein the body defines a holding area and a holding plane, and wherein the body includes a base and a bridge spaced from the base; attaching the fastening strap to the first segment of the elastic wrap; placing the second segment in the holding area; moving the tongue relative to the holding plane to engage the bridge; and securing at least a portion of the second segment between the tip and the bridge.

As used herein, "upward" and "downward" are used to the explain the relative movement of components only in relation to the orientation depicted and are not intended to otherwise limit or confine the possible movements of the components within the clip.

As used herein, "target material" means the elastic bandage wrap or other textile materials that are intended to be held in the clips of the present disclosure.

The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As recited herein, all numbers should be considered modified by the term "about".

As used herein, "a," "an," "the," "at least one," and "one or more" are used interchangeably. Thus, for example, a pressure pad comprising "a" protrusion can be interpreted as a pressure pad comprising "one or more" protrusions.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exhaustive list.

### Brief Description of the Drawings

The disclosure will be further described with reference to the drawings, wherein corresponding reference characters indicate corresponding parts throughout the several views, and wherein:
Figures 1A and 1B illustrate exemplary prior art clips.
Figure 2 is a top view of a clip according to one implementation of the present disclosure.
Figure 3 is a bottom view of the clip of Figure 2.
Figure 4 is a side view of the clip of Figure 2.
Figure 5 is a perspective view of a clip according to another implementation of the present disclosure.
Figure 6 illustrates a clip attached to an elastic wrap according to a method of the present disclosure.

While the above-identified figures set forth several embodiments of the disclosure, other embodiments are also contemplated, as noted in the description. In all cases, this disclosure presents the invention by way of representation and not limitation. It should be understood that numerous other modifications and embodiments can be devised by those skilled in the art, which fall within the scope of the invention.

### Detailed Description of Illustrative Embodiments

Figures 2-4 depict an implementation of a clip assembly 10 according to the present disclosure. The clip assembly 10 includes a body 20, a tongue 30, and a strap 40. The body 20 includes a base 21, first and second arms (22, 23), and a bridge 24, all cooperating to define a holding area 25. The first and second arms 22, 23 project from the base 21 and cooperate to define a holding plane. A bridge 24, depicted as having a trapezoidal shape, connects the first and second arms 22, 23. Alternatively, the connection bridge 24 may present a straight edge, such that the entire clip resembles a trapezoid. The clip body 20 may present any suitable shape, including square, polyhedral, circular, ovular, etc.

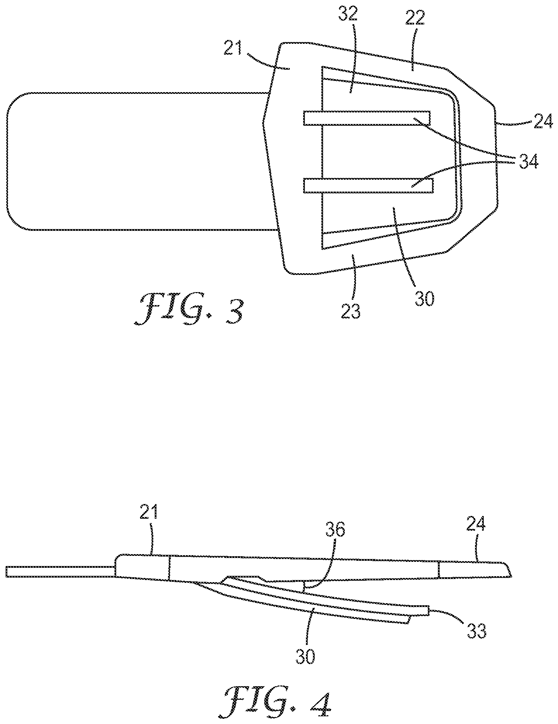

A tongue 30 projects from the base 21 in the direction of the bridge 24. The tongue 30 includes at least one engagement surface 31 and typically has a shape or profile similar to the holding area 25. As depicted in Figure 4, the tongue 30 can protrude at a downward, non-zero angle 36 relative to the holding plane. This protrusion angle 36 can assist in depressing the tongue relative to the holding plane (or holding plane relative to the tongue) as a target material is inserted into the holding area 25. In other embodiments, the tongue 30 may project at an upward, non-zero angle, may be coplanar with holding plane, or may project in parallel with the holding plane in the general direction of the bridge 24.

The tongue 30 includes a length dimension (as measured from base to tip 33) that is equal to or slightly greater than the length (as measured from base to bridge 24) of the holding area 25. The selection of a slightly greater length allows for a frictional interaction between the tip 33 and a portion of the bridge 24 when the tongue 30 is moved to engage the target material (according to methods of attachment described in more detail below). In use, the tip 33 is pressed in the direction of the holding plane to engage the bridge 24. A frictional interference fit between the tip 33 and the bridge 24 prevents the tongue 30 from returning to its original position relative to the holding plane. This interaction between the tip 33 and the bridge 24 also provides auditory and tactile feedback to the user that the clip 10 has been secured. Further, a portion of the target material may be held between the tip 33 and the bridge 24, with that interface acting as a pinch point for the material.

Generally, the tongue 30 has a width less than that of the holding area (as measured from first arm 22 to second arm 23), so that the tongue may more freely move between an engaged and non-engaged position relative to the bridge 24. In some implementations, the difference between such widths is at least 1/8 of an inch on each side, thus relative to each arm. In other implementations, the difference is at least ¼ of an inch on each side. In alternative implementations, securing the target material may rely at least partially on engagement between the side of the tongue and the first and/or second arm.

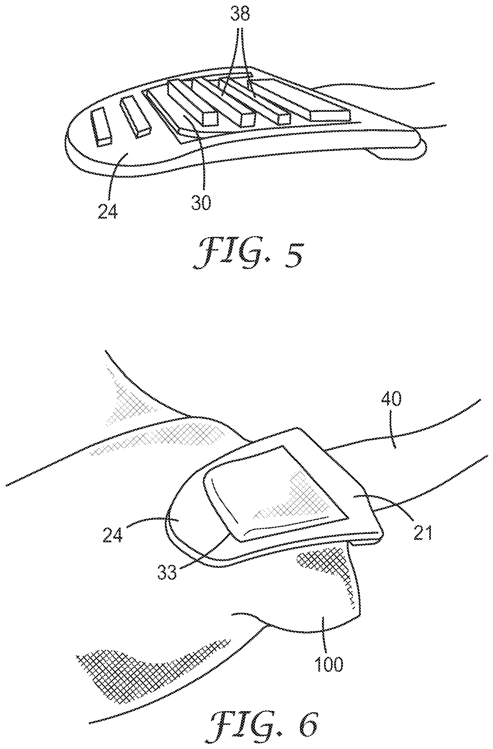

The material engagement surface 31 of the tongue 30 may be substantially smooth, as depicted in Figures 2-4. In other embodiments, such as the one depicted in Figure 5, the engagement surface may include one or more surface features 38. The surface features 38 may extend along substantially the entire engagement surface, or any portion thereof. Examples of suitable surface features can include, but are not limited to, a variety of polyhedral shapes, parallelepipeds, prismatoids, prismoids, etc., and combinations thereof. For example, the features can be polyhedral, conical, frusto-conical, pyramidal, frusto-pyramidal, spherical, partially spherical, hemispherical, ellipsoidal, dome-shaped, rail-shaped, cylindrical, and combinations thereof. The surface features 38 may, in certain implementations, provide an additional frictional stop to prevent the target material from sliding or otherwise moving undesirably while held in the clip 10.

As can be appreciated by reference to Figure 3, the surface 32 opposite the material engagement surface 31 can include support rails 34 or similar structure. The support rails 34 are typically designed to prevent excess movement of the tongue 30 relative to the holding plane, thereby retaining a bias toward the tongue's initial position. Further, the support rails 34 may prevent the inadvertent breaking or weakening of the tongue 30 due to excess force applied by the user, helping to ensure the clip assembly 10 is available for multiple uses. In other implementations, the opposite surface 32 can include the surface features 38 discussed above with respect to material engagement surface 31.

The body 20 of the clip can be made of several different types of material, including but not limited to metals and plastics. Particularly suitable plastics include nylon, polypropylene, and polycarbonate. The tongue 30 is preferably resilient and flexible enough to move relative to the body, but demonstrate a bias toward its original position. A clip 10 designed according to the present disclosure can potentially create approximately 7 lbf of holding force.

The body 20 of the clip may be coupled to a fastening strap 40, typically at the base 21. The base 21 may be coupled to a strap 40 by any known fixation mechanism, including, for example, welding and adhesion. The strap 40 typically comprises a flexible belt that can be secured to the target material or wrapped around a portion of the wearer's anatomy. The strap 40 is typically long enough to securely connect to the desired portions of the elastic wrap (or other target material) and prevent inadvertent removal.

The strap 40 typically comprises a relatively inelastic material (for example, a material having no more than about 30% stretch under tension) such as foam laminates (for example, a laminate including polyester inner layer, urethane foam, and nylon jersey for exterior durability) or a woven cotton or nylon strap. The strap 40 may also comprise a fastening surface similar, for example, to the hook in a "hook and loop application" applied to either side or both sides of the strap. The fastening surface can be directly applied to the target material. The hook and loop style fastening surface provides opportunity for peel/shear forces that can be modified based on the hook/loop pattern selected. In other implementations, the fastening surface includes adhesives (particularly pressure sensitive adhesives) and other series of microreplicated features (e.g., microstructures) that engage with the target material. Examples of pressure sensitive adhesives useful in the present disclosure include rubber-based adhesives (e.g., tackified natural rubbers, synthetic rubbers, and styrene block copolymers), (meth)acrylics (i.e., (meth)acrylates), poly(alpha-olefins), polyurethanes, and silicones. Other repositionable or removable adhesives can also be used on the fastening surface.

Alternatively, a clip assembly can be configured so that there is a clip body and corresponding tongue on both ends of the fastening strap. In other embodiments, the clip assembly includes two or more clip bodies connected in a multi-strap configuration.

The present disclosure also provides methods of attaching the clips described above to a target material, particularly an elastic bandage. In one implementation, the edge or other area of the target material 100 is folded over to create an edge having increased thickness. In alternative implementations, the target material 100 is placed in the clip body without significant alteration. The edge of the target material 100 is moved in the holding area in the general direction of the clip base 21. The user may choose to apply force to one of the tongue 30 or the body 20 to cause movement relative to the holding plane. Alternatively, the user may rely on the target material 100 to provide sufficient force as it travels towards the base 21. As the target material 100 nears the base 21 of the clip, or at any time prior, the user may move the tongue 30 relative to the holding plane in the direction of the bridge 24. Alternatively, the user may move the body (including the bridge) in the general direction of the tongue 30. In the embodiment depicted in Figure 6, the tongue 30 has been moved above the bridge 24, such that the surface opposite the material engagement surface rests on and frictionally engages the target material. The interaction between the tip 33 and bridge 24 provides an audible and tactile feedback that the clip is secured in place, as well as a pinch point for retain a portion of the target material. Once the clip is secured, the fastening strap 40 may be place on another section of the target material. Alternatively, the fastening strap 40 may be placed prior to inserting another segment into the holding area.

To secure an elastic bandage wrap around a portion of the anatomy, a user may attach either the fastening strap or the clip body to a segment of the elastic wrap. The user may then wrap material as much as desired or directed. Once the act of wrapping is completely, the user may secure the non-attached clip component or fastening surface to another segment of the wrap.

As a part of a kit, the clip may be provided attached to the target material (e.g., elastic bandage) or may be provided as a separate unit.

### Embodiments

1. A clip comprising: a body including a base, a first arm, a second arm, and a bridge, the body including a holding plane; a tongue projecting from the base in a direction relative to the holding plane; and a strap coupled to the body, wherein the tongue is operable to frictionally engage the bridge to secure a target material within the body.
2. The clip of claim 1, wherein the bridge connects the first and second arms at a location remote from the base.
3. The clip of claims 1 or 2, wherein the holding plane is at least partially defined by the first and second arms.
4. The clip of any of claim 1-3, wherein the tongue projects at a downward angle relative to the holding plane.
5. The clip of any of claims 1-3, wherein the tongue projects at an upward angle relative to the holding plane.
6. The clip of any of the previous claims, wherein the base, first arm, second arm, and bridge cooperate to define a holding area.
7. The clip of claim 6, wherein the tongue includes a length dimension that is at least equal to the length of the holding area.
8. The clip of claim 7, wherein the tongue includes a length dimension that is greater than the length of the holding area.
9. The clip of any of the previous claims, wherein an engagement between a tip of the tongue and the bridge prevents the tongue from returning to an initial position relative to the holding plane.
10. The clip of any of the previous claims, wherein a surface of the tongue includes a plurality of surface features projecting therefrom.
11. The clip of any of the previous claims, wherein the strap includes one or more fastening surfaces.
12. A method for securing a first segment of a target material to a second segment of the target material, the method comprising: providing a clip including a body, a tongue having a tip, and a fastening strap, wherein the body includes a holding area and a holding plane, and wherein the body further includes a base and a bridge spaced from the base; attaching the fastening strap to the first segment of the elastic wrap; placing the second segment in the holding area; moving the tongue relative to the holding plane to engage the bridge; and securing at least a portion of the second segment between the tip and the bridge.
13. A method for securing a first segment of a target material to a second segment of the target material, the method comprising: providing a clip including a body, a tongue having a tip, and a fastening strap, wherein the body includes a holding area and a holding plane, and wherein the body further includes a base and a bridge spaced from the base; attaching the fastening strap to the first segment of the elastic wrap; placing the at least a portion of the second segment in the holding area; moving the body relative to the holding plane to engage the tongue; and securing at least a portion of the second segment between the tip and the bridge.
14. The method of claim 12 or 13, wherein the bridge connects the first and second arms at a location remote from the base.
15. The method of any of the previous claims, wherein the holding plane is at least partially defined by the first and second arms.
16. The method of any of the previous claims, wherein the tongue projects at a downward angle relative to the holding plane.
17. The method of any one of claims 12-15, wherein the tongue projects at an upward angle relative to the holding plane.
18. The methods of any of the previous claims, wherein the base, first arm, second arm, and bridge cooperate to define a holding area.
19. The method of claim 18, wherein the tongue includes a length dimension that is at least equal to the length of the holding area.
20. The methods of claim 19, wherein the tongue includes a length dimension that is greater than the length of the holding area.
21. The methods of any the previous claims, wherein an engagement between a tip of the tongue and the bridge prevents the tongue from returning to the first position relative to the holding plane.
22. The methods of any of the previous claims, wherein a surface of the tongue includes a plurality of surface features projecting therefrom.
23. The methods of any of the previous claims, wherein the strap includes one or more fastening surfaces.
24. The method of any of the previous embodiments, wherein the target material is an elastic wrap.

It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A clip (10) comprising:
a body (20) including a base (21), a first arm (22) and a second arm (23) projecting from the base (21), and a bridge (24), the body (20) including a holding plane;
a tongue (30) projecting from the base (21) in a direction relative to the holding plane; and
a strap (40) coupled to the body (20), wherein the base (21), first arm (22), second arm (23), and bridge (24) cooperate to define a holding area (25), and wherein the tongue (30) includes a length dimension that is at least equal to the length of the holding area (25) such that the tongue (30) is operable to frictionally engage the bridge (24) to secure a target material within the body (20).

2. The clip (10) of claim 1, wherein the bridge (24) is connected to the first arm (22) at a location remote from the base (21).

3. The clip (10) of any of claim 1- 2, wherein the tongue (30) projects at a downward angle relative to the holding plane.

4. The clip (10) of any of claims 1 - 2, wherein the tongue (30) projects at an upward angle relative to the holding plane.

5. The clip (10) of any of claims 1 -2, wherein an engagement between a tip (33) of the tongue (30) and the bridge (24) prevents the tongue (30) from returning to an initial position relative to the holding plane.

6. The clip (10) of claim 1, wherein a surface (31) of the tongue (30) includes a plurality of surface features (38) projecting therefrom.

7. The clip (10) of claim 1, wherein the strap (40) includes one or more fastening surfaces.

8. A method for securing a first segment of a target material to a second segment of the target material, the method comprising:
providing the clip (10) of claim 1;
attaching the strap (40) to the first segment of an elastic wrap;
placing the at least a portion of the second segment in the holding area (25);
moving the body (20) relative to the holding plane to engage the tongue (30) or moving the tongue (30) relative to the holding plane to engage the bridge (24); and
securing at least a portion of the second segment between a tip (33) and the bridge (24).

9. The method of claim 8, wherein an engagement between the tip (33) of the tongue (30) and the bridge (24) prevents the tongue (30) from returning to the first position relative to the holding plane.

## Patentansprüche

1. Klammer (10), umfassend:
einen Körper (20), der eine Basis (21), einen ersten Arm (22) und einen zweiten Arm (23), die von der Basis (21) vorstehen, und eine Brücke (24) aufweist, wobei der Körper (20) eine Halteebene aufweist;
eine Zunge (30), die von der Basis (21) in einer Richtung in Bezug auf die Halteebene vorsteht; und
ein Band (40), das mit dem Körper (20) verbunden ist, wobei die Basis (21), der erste Arm (22), der zweite Arm (23) und die Brücke (24) zusammenwirken, um einen Haltebereich (25) zu definieren, und wobei die Zunge (30) eine Längenabmessung aufweist, die mindestens der Länge des Haltebereichs (25) entspricht, sodass die Zunge (30) funktionell in der Lage ist, mit der Brücke (24) in Reibungseingriff zu kommen, um ein Zielmaterial in dem Körper (20) zu sichern.

2. Klammer (10) nach Anspruch 1, wobei die Brücke (24) mit dem ersten Arm (22) an einer von der Basis (21) entfernten Position verbunden ist.

3. Klammer (10) nach einem der Ansprüche 1 bis 2, wobei die Zunge (30) in einem Abwärtswinkel in Bezug auf die Halteebene vorsteht.

4. Klammer (10) nach einem der Ansprüche 1 bis 2, wobei die Zunge (30) in einem Aufwärtswinkel in Bezug auf die Halteebene vorsteht.

5. Klammer (10) nach einem der Ansprüche 1 bis 2, wobei ein Eingriff zwischen einer Spitze (33) der Zunge (30) und der Brücke (24) verhindert, dass die Zunge (30) an eine Anfangsposition in Bezug auf die Halteebene zurückkehrt.

6. Klammer (10) nach Anspruch 1, wobei eine Oberfläche (31) der Zunge (30) mehrere Oberflächenmerkmale (38) aufweist, die davon vorstehen.

7. Klammer (10) nach Anspruch 1, wobei das Band (40) eine oder mehrere Befestigungsoberflächen aufweist.

8. Verfahren zum Sichern eines ersten Segments eines Zielmaterials an einem zweiten Segment des Zielmaterials, wobei das Verfahren Folgendes umfasst:
Bereitstellen der Klammer (10) nach Anspruch 1;
Befestigen des Bandes (40) an dem ersten Segment eines elastischen Verbands;
Anordnen des mindestens einen Abschnitts des zweiten Segments im Haltebereich (25);
Bewegen des Körpers (20) in Bezug auf die Halteebene, um die Zunge (30) in Eingriff zu bringen, oder Bewegen der Zunge (30) in Bezug auf die Halteebene, um die Brücke (24) in Eingriff zu bringen; und
Sichern von mindestens eines Abschnitts des zweiten Segments zwischen einer Spitze (33) und der Brücke (24).

9. Verfahren nach Anspruch 8, wobei ein Eingriff zwischen der Spitze (33) der Zunge (30) und der Brücke (24) verhindert, dass die Zunge (30) an die erste Position in Bezug auf die Halteebene zurückkehrt.

## Revendications

1. Attache (10) comprenant:
un corps (20) incluant une base (21), un premier bras (22) et un second bras (23) faisant saillie depuis la base (21) et un pont (24), le corps (20) incluant un plan de maintien ;
une langue (30) faisant saillie depuis la base (21) dans une direction relative au plan de maintien ; et
une sangle (40) couplée au corps (20), dans laquelle la base (21), le premier bras (22), le second bras (23) et le pont (24) coopèrent pour définir une zone de maintien (25), et dans laquelle la langue (30) inclut une dimension de longueur qui est au moins égale à la longueur de la zone de maintien (25) de sorte que la langue (30) puisse être actionnée de manière à venir en prise par friction avec le pont (24) pour fixer un matériau cible à l'intérieur du corps (20).

2. Attache (10) selon la revendication 1, dans laquelle le pont (24) est relié au premier bras (22) au niveau d'un emplacement éloigné de la base (21).

3. Attache (10) selon l'une quelconque des revendications 1 à 2, dans laquelle la langue (30) fait saillie selon un angle dirigé vers le bas par rapport au plan de maintien.

4. Attache (10) selon l'une quelconque des revendications 1 à 2, dans laquelle la langue (30) fait saillie selon un angle dirigé vers le haut par rapport au plan de maintien.

5. Attache (10) selon l'une quelconque des revendications 1 à 2, dans laquelle une mise en prise entre une pointe (33) de la langue (30) et le pont (24) empêche la langue (30) de revenir à une position initiale par rapport au plan de maintien.

6. Attache (10) selon la revendication 1, dans laquelle une surface (31) de la langue (30) inclut une pluralité d'éléments de surface (38) faisant saillie depuis celle-ci.

7. Attache (10) selon la revendication 1, dans laquelle la sangle (40) inclut une ou plusieurs surfaces d'attache.

8. Procédé pour la fixation d'un premier segment de matériau cible à un second segment du matériau cible, le procédé comprenant :
la fourniture de l'attache (10) selon la revendication 1 ;
la fixation de la sangle (40) au premier segment d'une bande élastique ;
le placement de l'au moins une partie du second segment dans la zone de maintien (25) ;
le déplacement du corps (20) par rapport au plan de maintien afin de venir en prise avec la langue (30) ou le déplacement de la langue (30) par rapport au plan de maintien de manière à venir en prise avec le pont (24) ; et
la fixation d'au moins une partie du second segment entre une pointe (33) et le pont (24).

9. Procédé selon la revendication 8, dans lequel une mise en prise entre la pointe (33) de la langue (30) et le pont (24) empêche la langue (30) de revenir à la première position par rapport au plan de maintien.
